(19) Europäisches Patentamt / European Patent Office / Office européen des brevets

(11) **EP 2 586 758 B1**

(12) **EUROPEAN PATENT SPECIFICATION**

(45) Date of publication and mention
of the grant of the patent:
**21.10.2015 Bulletin 2015/43**

(21) Application number: **11798219.9**

(22) Date of filing: **23.06.2011**

(51) Int Cl.:
*C07C 17/23* (2006.01)       *C07C 21/18* (2006.01)
*C07B 61/00* (2006.01)

(86) International application number:
**PCT/JP2011/064424**

(87) International publication number:
**WO 2011/162338 (29.12.2011 Gazette 2011/52)**

(54) **METHOD FOR MANUFACTURING 2,3,3,3-TETRAFLUOROPROPENE**

VERFAHREN ZUR HERSTELLUNG VON 2,3,3,3-TETRAFLUORPROPEN

PROCÉDÉ DE FABRICATION DE 2,3,3,3-TÉTRAFLUOROPROPÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2010 JP 2010142666**

(43) Date of publication of application:
**01.05.2013 Bulletin 2013/18**

(73) Proprietor: **Asahi Glass Company, Limited
Tokyo 100-8405 (JP)**

(72) Inventors:
• **KAWAGUCHI, Satoshi
Tokyo 100-8405 (JP)**
• **OKAMOTO, Hidekazu
Tokyo 100-8405 (JP)**
• **TAKEUCHI, Yu
Tokyo 100-8405 (JP)**
• **TAKAGI, Hirokazu
Tokyo 100-8405 (JP)**
• **WATANABE, Kunio
Tokyo 100-8405 (JP)**
• **YANASE, Koichi
Tokyo 100-8405 (JP)**

(74) Representative: **Müller-Boré & Partner
Patentanwälte PartG mbB
Friedenheimer Brücke 21
80639 München (DE)**

(56) References cited:
**WO-A2-2008/060614       WO-A2-2009/035130
JP-A- 2 286 635       JP-A- 2010 510 221**

• **'Kagaku Daijiten 2', 1960, KYORITSU SHUPPAN CO., LTD., page 437**
• **'Kogyo Hanno Sochi -Sentei.Sekkei.Jitsurei', 1984, BAIFUKAN CO., LTD., page 21**

**Description**

TECHNICAL FIELD

The present invention relates to a process for producing 2,3,3,3-tetrafluoropropene.

BACKGROUND ART

2,3,3,3-tetrafluoropropene ($CF_3CF=CH_2$, HFO-1234yf) contains no chlorine and thus is useful as an alternative compound for chlorofluorocarbons to be used for e.g. refrigerants.

As a process for producing HFO-1234yf, a process may, for example, be mentioned wherein 1,1-dichloro-2,2,3,3,3-pentafluoropropane ($CF_3CF_2CHCl_2$, HCFC-225ca) is subjected to a dehydrofluorination reaction to obtain 1,1-dichloro-2,3,3,3-tetrafluoropropene ($CF_3CF=CCl_2$, CFO-1214ya), and then, CFO-1214ya is reacted with hydrogen and reduced to obtain HFO-1234yf.

As a method of reducing CFO-1214ya to obtain HFO-1234yf, the following method (i) may, for example, be mentioned.

(i) A method for obtaining HFO-1234yf by reacting a raw material compound composed of CFO-1214ya and hydrogen in the presence of a catalyst having palladium supported on alumina so that the ratio of the number of moles of the hydrogen to the number of moles of chlorine atoms in the raw material compound ($H_2$/Cl) will be from 0.5 to 2 (Patent Document 1).

However, the method (i) has a problem such that together with HFO-1234yf, an excessively reduced product 1,1,1,2-tetrafluoropropane ($CF_3CHFCH_3$, HFC-254eb) will be formed as a by-product in a large amount.

PRIOR ART DOCUMENT

PATENT DOCUMENT

Patent Document 1: WO2008/060614

DISCLOSURE OF INVENTION

TECHNICAL PROBLEM

It is an object of the present invention to provide a process for producing 2,3,3,3-tetrafluoropropene (HFO-1234yf), whereby it is possible to suppress formation of an excessively reduced product HFC-254eb as a by-product.

SOLUTION TO PROBLEM

In order to solve the above problem, the present invention has adopted the following construction.

[1] A process for producing 2,3,3,3-tetrafluoropropene, which comprises reacting a raw material compound composed of at least one of 1,1-dichloro-2,3,3,3-tetrafluoropropene and 1-chloro-2,3,3,3-tetrafluoropropene, and hydrogen, in a gas phase in the presence of a catalyst, wherein the catalyst is a palladium catalyst supported on active carbon, and the ratio of the hydrogen to the raw material compound to be introduced to a catalyst layer packed with the palladium-supporting active carbon is at most 0.7 as represented by the ratio of the number of moles of the hydrogen to the number of moles of chlorine atoms in the raw material compound ($H_2$/Cl).

[2] The process for producing 2,3,3,3-tetrafluoropropene according to the above [1], wherein the supported amount of palladium in the palladium-supporting active carbon is from 0.1 to 10 mass% based on the active carbon.

[3] The process for producing 2,3,3,3-tetrafluoropropene according to the above [1] or [2], wherein the active carbon is coconut shell active carbon.

[4] The process for producing 2,3,3,3-tetrafluoropropene according to any one of the above [1] to [3], wherein the ratio of the number of moles of the hydrogen to the number of moles of chlorine atoms in the raw material compound ($H_2$/Cl) is from 0.1 to 0.6.

[5] The process for producing 2,3,3,3-tetrafluoropropene according to any one of the above [1] to [4], wherein the raw material compound is 1,1-dichloro-2,3,3,3-tetrafluoropropene, or a mixture of 1,1-dichloro-2,3,3,3-tetrafluoropropene and 1-chloro-2,3,3,3-tetrafluoropropene wherein the proportion of 1,1-dichloro-2,3,3,3-tetrafluoropropene to the total number of moles of both is at least 50 mol%.

[6] The process for producing 2,3,3,3-tetrafluoropropene according to any one of the above [1] to [5], wherein the 1-chloro-2,3,3,3-tetrafluoropropene is a compound formed as a byproduct in the production of 2,3,3,3-tetrafluoropropene by a reaction of 1,1-dichloro-2,3,3,3-tetrafluoropropene with hydrogen.

[7] The process for producing 2,3,3,3-tetrafluoropropene according to any one of the above [1] to [6], wherein the raw material compound and hydrogen are introduced to a gas inlet portion of the catalyst layer, and hydrogen is introduced from at least one position between the gas inlet portion and a gas outlet portion of the catalyst layer, and wherein the ratio of the total amount of hydrogen including the hydrogen to be introduced to the gas inlet portion of the catalyst layer to the raw material compound is the above-mentioned ratio in the number of moles.

[8] A process for producing 2,3,3,3-tetrafluoropropene, which comprises introducing a raw material compound composed of 1,1-dichloro-2,3,3,3-tetrafluoropropene, or 1,1-dichloro-2,3,3,3-tetrafluoropropene and 1-chloro-2,3,3,3-tetrafluoropropene, and hydrogen in such an amount that the ratio of the number of moles of hydrogen to the number of moles of chlorine atoms in the raw material compound ($H_2/Cl$) becomes at most 0.7, to a catalyst layer packed with a palladium catalyst supported on active carbon, to subject them to a gas phase reaction to form 2,3,3,3-tetrafluoropropene, and separating the formed 2,3,3,3-tetrafluoropropene from byproduct 1-chloro-2,3,3,3-tetrafluoropropene to isolate the 2,3,3,3-tetrafluoropropene, wherein recycling of 1-chloro-2,3,3,3-tetrafluoropropene is carried out at least once, said recycling comprising blending the byproduct 1-chloro-2,3,3,3-tetrafluoropropene with 1,1-dichloro-2,3,3,3-tetrafluoropropene to form a fresh raw material compound and carrying out its reaction with the hydrogen.

[9] The process for producing 2,3,3,3-tetrafluoropropene according to the above [8], wherein the supported amount of palladium in the palladium-supporting active carbon is from 0.1 to 10 mass% based on the active carbon.

[10] The process for producing 2,3,3,3-tetrafluoropropene according to the above [8] or [9], wherein hydrogen is introduced in such an amount that the ratio of the number of moles of the hydrogen to the number of moles of chlorine atoms in the raw material compound ($H_2/Cl$) becomes from 0.1 to 0.6, to the catalyst layer.

ADVANTAGEOUS EFFECT OF INVENTION

According to the process of the present invention, it is possible to suppress formation of an excessively reduced product HFC-254eb as a by-product.

BRIEF DESCRIPTION OF DRAWING

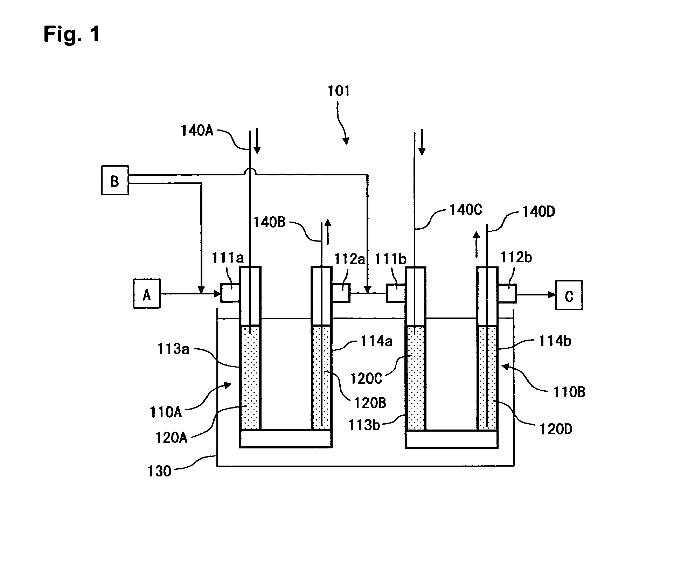

Fig. 1 is a schematic view illustrating a reaction apparatus used in Examples.

DESCRIPTION OF EMBODIMENT

In the process for producing 2,3,3,3-tetrafluoropropene (HFO-1234yf) of the present invention, a raw material compound composed of at least one of 1,1-dichloro-2,3,3,3-tetrafluoropropene ($CF_3CF=CCl_2$, CFO-1214ya) and 1-chloro-2,3,3,3-tetrafluoropropene ($CF_3CF=CHCl$, HCFO-1224yd), and hydrogen, are reacted in the presence of a catalyst. That is, the process of the present invention is a process to obtain HFO-1234yf by subjecting the raw material compound and hydrogen to a gas phase reaction in the presence of a catalyst. Further, a gas comprising the raw material compound and the hydrogen will hereinafter sometimes be referred to as the raw material mixed gas.

CFO-1214ya and HCFO-1224yd will form HFO-1234yf by the reactions represented by the following formulae (1) and (2), respectively.

$$CF_3CF=CCl_2 + 2H_2 \rightarrow CF_3CF=CH_2 + 2HCl \qquad (1)$$

$$CF_3CF=CHCl + H_2 \rightarrow CF_3CF=CH_2 + HCl \qquad (2)$$

For the reaction of the raw material compound and the hydrogen, specifically, a method may, for example, be mentioned wherein a catalyst layer is formed by packing a palladium catalyst-supporting active carbon in a reactor, and to such a catalyst layer, the raw material mixed gas is introduced and reacted.

The catalyst in the process of the present invention is a palladium catalyst, and such a palladium catalyst is used as supported on active carbon. The palladium catalyst may be not only palladium simple substance but also a palladium alloy. Further, it may be a mixture of palladium with another metal, or a composite catalyst having palladium and another metal separately supported on carriers. A palladium alloy catalyst may, for example, be a palladium/platinum alloy catalyst or a palladium/rhodium alloy catalyst.

The active carbon may be one prepared by using, as a raw material, wood, charcoal, fruit shell, coconut shell, peat, lignite, coal or the like, and one obtained from a plant raw material rather than from a mineral raw material is preferred. Particularly preferred is coconut shell active carbon.

As the shape of the carrier, formed coal having a length of from about 2 to 5 mm, pulverized coal of from about 4 to 50 mesh or granular coal may, for example, be mentioned. Among them, pulverized coal of from 4 to 20 mesh or formed coal is preferred.

The supported amount of palladium in the palladium-supporting active carbon is, based on active carbon, preferably from 0.1 to 10 mass%, more preferably from 0.5 to 1 mass%. When the supported amount of palladium is at least the lower limit value, the conversion of the raw material compound and the hydrogen will be improved. When the above supported amount of palladium is at most the upper limit value, an excessive temperature rise of the catalyst layer by a heat of reaction can easily be controlled, and formation of by-products can easily be reduced.

Further, the active carbon having palladium supported thereon may further have a metal other than palladium supported thereon.

The metal other than palladium may, for example, be a Group 8 element such as iron, ruthenium or osmium; a Group 9 element such as cobalt, rhodium or iridium; a Group 10 element such as nickel or platinum; or gold. Such metals other than palladium may be used alone, or two or more of them may be used in combination.

The proportion of the metal other than palladium is preferably from 0.01 to 50 parts by mass, per 100 parts by mass of palladium. The composite catalyst having the metal other than palladium supported together with palladium tends to have higher catalyst durability than the catalyst having only palladium supported alone.

In the present invention, the catalyst layer is formed by packing the palladium-supporting active carbon in a reactor. The packed density of the palladium-supporting active carbon in the catalyst layer is preferably from 0.5 to 1 $g/cm^3$, more preferably from 0.6 to 0.8 $g/cm^3$. When the packed density of the palladium-supporting active carbon is at least the lower limit value, the packed amount of the palladium-supporting active carbon per unit volume is large, whereby the amount of gas to be reacted can be increased, and the productivity will be improved. When the packed density of the palladium-supporting active carbon is at most the upper limit value, the temperature rise of the catalyst layer due to a heat of reaction can easily be controlled, and it becomes easy to suppress the formation of by-products.

In the reactor, there may be one or more portions packed with the palladium-supporting active carbon.

With a view to suppressing formation of the excessively reduced product HFC-254eb as a by-product, the proportion of the hydrogen to the raw material compound to be introduced to the catalyst layer is adjusted to be at most 0.7 as represented by the ratio of the number of moles of the hydrogen to the number of moles of chlorine atoms in the raw material compound ($H_2$/Cl). Such a ratio ($H_2$/Cl) should better be smaller, whereby formation of HFC-254eb can easily be suppressed, and it is preferably at most 0.6, more preferably at most 0.5. Further, the ratio ($H_2$/Cl) is preferably at least 0.1, more preferably at least 0.2, from the viewpoint of the yield of HFO-1234yf.

In a case where the hydrogen is introduced dividedly as will be described hereinafter, the ratio of the total amount of hydrogen to be introduced to the catalyst layer to the raw material compound to be introduced to the catalyst layer is likewise preferably adjusted to be at most 0.7, more preferably at most 0.6, further preferably at most 0.5 by the above ratio in the number of moles ($H_2$/Cl). Further, the ratio ($H_2$/Cl) is preferably at least 0.1, more preferably at least 0.2.

For the gas phase reaction, the temperature of the catalyst layer is a temperature exceeding the dew point of the raw material mixed gas. Since the boiling point of CFO-1214ya is 46°C, and the boiling point of HCFO-1224yd is assumed to be from 4 to 10°C and in view of the reactivity, it is preferably at least 50°C. In order to further increase the conversion, it is more preferably at least 60°C, particularly preferably at least 80°C.

There is a problem such that the temperature of the catalyst layer gradually decreases along with the progress of deterioration of the catalyst, whereby the conversion decreases. Therefore, it is preferred to carry out an operation to maintain the temperature of the catalyst layer at a sufficient temperature, so that a high conversion can be maintained. For example, in a case where the temperature is maintained by heating the catalyst layer from outside by e.g. a heating medium, it is possible to increase the temperature of the catalyst layer by gradually increasing the temperature of the heating medium.

Here, the temperature of the catalyst layer is a temperature of the catalyst layer which can be maintained by heating from outside. Usually, the raw material mixed gas is reacted at a region of the catalyst layer, and by the generation of a reaction heat, the reaction zone (the region where the raw material mixed gas is reacted) becomes a higher temperature than other regions of the catalyst layer. The catalyst activity in this reaction zone gradually decreases as the time passes, and usually, the reaction zone gradually moves from the inlet portion of the raw material mixed gas to the downstream side in the gas stream direction. Further, on the downstream side of the reaction zone, a formed gas having a high temperature formed in the reaction zone will flow and usually becomes a higher temperature than the temperature of the catalyst layer, and the temperature gradually lowers as the distance from the reaction zone increases. The temperature of the catalyst layer of the present invention is meant for the temperature on the upstream side of the reaction zone i.e. the temperature of the catalyst layer, of which the temperature is maintained by heating from outside by e.g. a heating medium.

Further, in the process of the present invention, the maximum temperature of the catalyst layer is maintained to be preferably at most 130°C, more preferably at most 100°C during the reaction with a view to suppressing formation of a by-product. That is, in the process of the present invention, it is preferred to prevent an excessive temperature rise of the catalyst layer due to the heat of reaction and to adjust the maximum temperature of the catalyst layer to be at most the above upper limit value. As mentioned above, the temperature at the reaction zone where the raw material is reacted and at a region on the downstream side thereof becomes higher than the temperature of other regions of the catalyst layer due to the heat of reaction. The maximum temperature of the catalyst layer during the reaction is meant for the maximum temperature in the region of the catalyst layer where the temperature becomes higher than other regions due to the generation of such a heat of reaction.

As a method for measuring the maximum temperature of the catalyst layer during the reaction, the following measuring method employing an insertion-type thermometer may, for example, be mentioned.

In the reaction of the raw material compound and the hydrogen in the catalyst layer, firstly, the catalyst at the gas inlet portion contributes to the reaction, and as the catalyst at the gas inlet portion deteriorates, the catalyst on the downstream side thereof will contribute to the reaction, and in such a manner, the reaction zone in the catalyst layer gradually moves towards to the gas outlet side. That is, the portion showing the maximum temperature of the catalyst layer moves along with the movement of the reaction zone of the raw material compound and the hydrogen. Accordingly, by preliminarily positioning the measuring portion of the insertion-type thermometer at the gas inlet portion of the catalyst layer and moving the measuring portion along with the progress of the reaction, the maximum temperature of the catalyst layer can be measured.

As a method to maintain the maximum temperature of the catalyst layer during the reaction to be at most the above upper limit value, a method (method ($\alpha$)) of introducing the hydrogen dividedly to the catalyst layer is preferred from such a viewpoint that the productivity can easily be maintained to be high while controlling the maximum temperature of the catalyst layer to be low. If the hydrogen is introduced dividedly to plural portions of the catalyst layer, it is possible to disperse the reaction zones of the catalyst layer without changing the amount of the raw material compound to be introduced, whereby generation of the heat of reaction is not localized at one portion. Therefore, it is possible to easily suppress local excessive heat generation in the catalyst layer, without lowering the productivity.

Divided introduction of the hydrogen means that the raw material compound and the hydrogen are introduced to the gas inlet portion of the catalyst layer, and at the same time, hydrogen is introduced from at least one portion between the gas inlet portion and the gas outlet portion of the catalyst layer. That is, hydrogen is introduced from at least one portion of the catalyst layer in addition to the inlet portion where the raw material mixed gas is introduced, i.e. from a total of at least two portions.

Specifically, the raw material mixed gas to be introduced to the gas inlet portion of the catalyst layer (the gas inlet portion on the most upstream side in the gas flow direction) is a mixed gas comprising a part of hydrogen and all amount of the raw material compound to be introduced to the catalyst layer. The rest of hydrogen is introduced to the catalyst layer on the downstream side in the gas flow direction to mix the hydrogen to the gas flowing in the catalyst layer at the introduction position (usually formed gas after a part of the raw material compound was reacted with hydrogen), so that the hydrogen is reacted with an unreacted raw material compound in the catalyst layer on the downstream side from the introduction position of the hydrogen, and the formed gas is discharged from the outlet of the catalyst layer (the gas outlet portion on the most downstream side in the gas flow direction). It is preferred that at least a part of the hydrogen in the raw material mixed gas is reacted with the raw material compound between the inlet portion of the raw material mixed gas and the next introduction portion of hydrogen. Further, it is preferred that the hydrogen introduction portion on the most downstream side in the gas flow direction is provided at a location where the introduced hydrogen and the raw material compound are sufficiently reacted in the catalyst layer between the hydrogen introduction portion and the gas outlet portion.

Introduction of the hydrogen in the method ($\alpha$) may be divided into two portions or divided into three or more portions. It is preferred to divide the introduction into two portions, whereby the process can be simplified.

The divided proportions of the hydrogen to be introduced dividedly to at least two portions in the catalyst layer are preferably such that the respective gas amounts divided are equal amounts, whereby it is easy to maintain the maximum temperature of the catalyst layer to be low.

In a case where there are two or more portions packed with the catalyst-supporting active carbon in the reactor, the divided introduction of the hydrogen may, for example, be carried out by a method wherein a part of the hydrogen is introduced together with the raw material compound to the first stage packed portion, and the rest is introduced to the second and subsequent stage packed portions.

Further, as a method for controlling the maximum temperature of the catalyst layer other than the method ($\alpha$), a method (method ($\beta$)) of letting an inert gas flow in the catalyst layer together with the raw material compound and the hydrogen, may be mentioned. By adjusting the concentration of the raw material compound and the hydrogen flowing in the catalyst layer by letting the inert gas flow, it is possible to suppress an excessive temperature rise of the catalyst layer by a heat of reaction. Further, it is possible to use a diluent gas other than an inert gas instead of the inert gas or together with the inert gas.

As such an inert gas, nitrogen, rare gases or chlorofluorocarbons inert to the hydrogenation reaction may, for example, be mentioned. The diluent gas other than the inert gas may, for example, be hydrogen chloride.

The amount of the inert gas to be introduced to the catalyst layer is preferably at least 0.1 mol, more preferably at least 0.5 mol, per 1 mol of the raw material compound, from such a viewpoint that it is thereby easy to maintain the maximum temperature of the catalyst layer to be low, to reduce formation of by-products and to suppress deterioration of the catalyst. Further, the amount of the inert gas to be introduced is preferably at most 10 mol, more preferably at most 4 mol, per 1 mol of the raw material compound, from the viewpoint of the recovery rate of the inert gas.

Further, as a method for controlling the maximum temperature of the catalyst layer other than the method ($\alpha$) and the method ($\beta$), a method (method ($\gamma$)) may be mentioned wherein the temperature of the catalyst layer is made lower, while the lower limit is the dew point of the raw material mixed gas. By keeping the temperature of the catalyst layer to be low, a more swift heat removal of the heat of reaction becomes possible, and an excessive temperature rise of the catalyst layer can be suppressed.

In the method ($\gamma$), the temperature of the catalyst layer is preferably adjusted to be higher than the dew point and lower than 50°C, since the catalyst layer being at a lower temperature is advantageous to suppress formation of a byproduct which is hardly separable from HFO-1234yf, and since in the reaction in a state where the raw material is liquefied, the yield of HFO-1234yf deteriorates due to an increase of the formation of a byproduct by excessive reduction of HFO-1234yf. It is more preferably higher than the dew point and lower than 30°C.

For the control of the maximum temperature of the catalyst layer, the method ($\alpha$), the method ($\beta$) or the method ($\gamma$) may preferably be used alone, or two or three of them may preferably be used in combination.

The reaction pressure is preferably atmospheric pressure from the viewpoint of the operation efficiency.

The contact time of the raw material compound to the catalyst is preferably from 4 to 60 seconds, more preferably from 8 to 40 seconds. This contact time is a contact time of the raw material compound gas calculated from the amount of the gas to be introduced to the reactor and the volume of the catalyst layer.

In the process of the present invention, the linear velocity u of the raw material compound represented by the following formula (I) in the catalyst layer, varies depending upon the diameter of the reaction tube, but in the case of the diameter of the reaction tube which is commonly used for a gas phase reduction reaction, it is preferably from 0.1 to 100 cm/sec., more preferably from 1 to 30 cm/sec. This linear velocity u is a linear velocity of the raw material compound calculated from the amount of the gas to be introduced to the reactor and the volume of the catalyst layer. When the linear velocity u of the raw material compound is at least the lower limit value, the productivity will be improved. Especially when the gas linear velocity is at least 1 cm/sec., the gas is likely to flow uniformly in the catalyst layer. When the linear velocity u of the raw material compound is at most the upper limit, the conversion of the raw material compound and the hydrogen will be improved. Especially when the gas linear velocity is at most 30 cm/sec., the temperature control in the vicinity of the reaction point due to heat generation will be easy.

$$u=(W/100)\times V/S \quad (I)$$

In the formula (I), W is the concentration (mol%) of the raw material compound in the entire gas flowing through the catalyst layer, V is the flow rate ($cm^3$/sec) of the entire gas flowing through the catalyst layer, and S is the cross-sectional area ($cm^2$) of the catalyst layer to the flow direction of the gas.

As a reactor to be used for the process of the present invention, a known reactor capable of forming a catalyst layer having a catalyst-supporting carrier packed, may be mentioned.

As the material for the reactor, glass, iron, nickel or an alloy containing such a metal as the main component may, for example, be mentioned.

The formed gas after the reaction contains, in addition to the desired product HFO-1234yf, an unreacted raw material, HCFO-1224yd formed as a reaction intermediate and HCl.

HCl contained in the formed gas can be removed by blowing the formed gas into an alkali aqueous solution for its neutralization. The alkali to be used for such an alkali aqueous solution may, for example, be sodium hydroxide or potassium hydroxide.

As a method for recovering HFO-1234yf from the formed gas, a known method such as distillation may, for example, be employed.

The raw material compound is a gas composed of at least one of CFO-1214ya and HCFO-1224yd.

CFO-1214ya can be produced by a known method. For example, a method may be mentioned wherein 1,1-dichloro-2,2,3,3,3-pentafluoropropane ($CHCl_2CF_2CF_3$, HCFC-225ca) is subjected to a dehydrofluorination reaction by contacting it with an alkali aqueous solution in the presence of a phase-transfer catalyst. For such a reaction, dichloropentafluoropropane (HCFC-225) including HCFC-225ca may be used, and only HCFC-225ca in HCFC-225 may selectively be dehydrofluorinated by the above phase-transfer catalyst. After the reaction, CFO-1214ya can be separated and recovered by a known method such as distillation.

The above HCFC-225 including HCFC-225ca can be produced by reacting tetrafluoroethylene and dichlorofluoromethane in the presence of a catalyst such as aluminium chloride. The HCFC-225 obtainable by such a reaction contains HCFC-225ca and 1,3-dichloro-1,2,2,3,3-pentafluoropropane ($CHClFCF_2CClF_2$, HCFC-225cb) as the main components, and further contains a small amount of 2,2-dichloro-1,1,3,3,3-pentafluoropropane ($CHF_2CCl_2CF_3$, HCFC-225aa), 2,3-dichloro-1,1,2,3,3-pentafluoropropane ($CHF_2CClFCClF_2$, HCFC-225bb), etc.

As such HCFC-225 including HCFC-225ca, a commercial product may be employed. As such a commercial product, ASAHIKLIN AK225 (tradename, manufactured by Asahi Glass Company, Limited, mixture of 48 mol% of HCFC-225ca and 52 mol% of HCFC-225cb) may, for example, be mentioned.

As the above phase-transfer catalyst, tetrabutylammonium bromide (TBAB) is preferred.

HCFO-1224yd is formed as an intermediate at the time of obtaining HFO-1234yf by reacting CFO-1214ya with hydrogen.

HCFO-1224yd recovered from the formed gas after the reaction may be reacted, as the raw material compound together with CFO-1214ya, with the hydrogen, or separately from CFO-1214ya, HCFO-1224yd may alone be reacted with the hydrogen.

In a case where a mixture of CFO-1214ya and HCFO-1224yd is used as the raw material compound, usually a mixture having a small proportion of HCFO-1224yd is used, since HCFO-1224yd is an intermediate at the time of obtaining HFO-1234yf from the above CFO-1214ya. Thus, the proportion of HCFO-1224yd based on the total amount of CFO-1214ya and HCFO-1224yd is preferably at most 50 mol%, more preferably at most 25 mol%.

According to the process of the present invention as described in the foregoing, by means of a catalyst having palladium supported on active carbon, the raw material compound and the hydrogen are introduced to the catalyst layer in such a proportion that the ratio of the number of moles of the hydrogen to the number of moles of chlorine atoms in the raw material compound ($H_2/Cl$) will be at most 0.7, and subjected to a gas phase reaction, whereby formation of the excessively reduced product HFC-254eb as a by-product can be suppressed. Consequently, the amounts of the desired product HFO-1234yf and HCFO-1224yd convertible to HFO-1234yf will increase, whereby it is possible to efficiently produce highly pure HFO-1234yf.

EXAMPLES

Now, the present invention will be described in further detail with reference to Examples and Comparative Examples. However, it should be understood that the present invention is by no means restricted by the following description. Examples 1 to 3 are working Examples of the present invention and Examples 4 and 5 are Comparative Examples.

[EXAMPLE 1]

For the production of HFO-1234yf ($CF_3CF=CH_2$), a reaction apparatus 101 shown in Fig. 1 was used.

As shown in Fig. 1, the reaction apparatus 101 is provided with two reaction tubes 110A and 110B and an oil bath 130 for immersion of such reaction tubes 110A and 110B. The reaction tube 110A has catalyst-packing portions 113a and 114a at two positions on the inlet 111a side and the outlet 112a side. Likewise, the reaction tube 110B has catalyst-packing portions 113b and 114b at two positions on the inlet 111b side and the outlet 112b side. The outlet 112a of the reaction tube 110A is connected by piping to the inlet 111 b of the reaction tube 110B.

As the reaction tubes 110A and 110B, reaction tubes made of Inconel (registered trademark) 600 and having an inner diameter of 2.54 cm and a length of 100 cm, were used. Further, using a palladium-supporting active carbon having 0.5 mass% of palladium supported on coconut shell active carbon having a particle size of from 4 to 8 mesh, such palladium-supporting active carbon was packed in the catalyst-packing portions 113a and 114a of the reaction tube 110A to form a catalyst layer 120A and a catalyst layer 120B each having a height of 40 cm. Likewise, the above palladium-supporting active carbon was packed in the respective catalyst-packing portions 113b and 114b of the reaction tube 110B to form a catalyst layer 120C and a catalyst layer 120D each having a height of 40 cm. The packed density of the palladium-supporting active carbon in catalyst layers 120A to 120D was adjusted to be 0.73 $g/cm^3$.

Then, the reaction tube 110A and the reaction tube 110B were immersed in the oil bath 130 so that all of the catalyst layers 120A to 120D were immersed, and the catalyst layers 120A to 120D were heated to 80°C.

Then, a raw material compound (A) having CFO-1214ya ($CF_3CF=CCl_2$) and HCFO-1224yd ($CF_3CF=CHCl$) mixed in a ratio of 6:1, and hydrogen (B) were permitted to flow through reaction tubes 110A and 110B to obtain formed gas (C).

The contact time of the raw material compound (A) to the catalyst layers 120A to 120D was adjusted to be 23 seconds, and the ratio of the number of moles of the total introduction amount of the hydrogen (B) to be introduced to the catalyst layers to the number of moles of chlorine atoms in the raw material compound (A) ($H_2/Cl$) was adjusted to be 0.43. The linear velocity u of the raw material compound (A) was adjusted to be 7 cm/sec.

Further, with respect to the hydrogen (B), 50% of the total introduced amount was introduced from the inlet 111a of the reaction tube 110A together with the raw material compound (A), and the rest of 50% was introduced to the piping portion connecting the reaction tube 110A and the reaction tube 110B. That is, in the catalyst layer having a length of 160 cm and consisting of catalyst layers 120A to 120D, the hydrogen (B) was dividedly introduced at two portions i.e. the catalyst layer 120A (0 cm point) and the catalyst layer 120C (80 cm point).

Further, the maximum temperature of the catalyst layers 120A to 120D during the reaction was measured by insertion-type thermometers 140A to 140D inserted respectively to such catalyst layers and found to be at most 100°C.

[EXAMPLE 2 and 3]

Formed gas (C) was obtained in the same manner as in Example 1 except that the ratio ($H_2$/Cl) was changed as shown in Table 1.

[EXAMPLE 4]

Formed gas (C) was obtained in the same manner as in Example 1 except that the palladium-supporting active carbon was changed to a palladium-supporting alumina having 0.5 mass% of palladium supported on alumina, and the ratio ($H_2$/Cl) was changed as shown in Table 1.

[EXAMPLE 5]

Formed gas (C) was obtained in the same manner as in Example 1 except that the ratio ($H_2$/Cl) was changed as shown in Table 1.

[Evaluation method]

The formed gas (C) thus obtained was analyzed by gas chromatography (GC), and the conversion ratio X (unit: %) to HFO-1234yf, the conversion ratio Y (unit: %) to HCFO-1224yd, and the conversion ratio Z (unit: %) to HFC-254eb ($CF_3CHFCH_3$) were calculated by the following formulae (II) to (IV), respectively.

$$X=[c/((a+b)/2)]\times100 \quad (II)$$

$$Y=[d/((a+b)/2)]\times100 \quad (III)$$

$$Z=[e/((a+b)/2)]\times100 \quad (IV)$$

(In the above formulae, a represents the number of moles of CFO-1214ya in the raw material compound (A), b the number of moles of HCFO-1224yd in the raw material compound (A), c the number of moles of HFO-1234yf in the formed gas (C), d the number of moles of HCFO-1224yd in the formed gas (C) and e the number of moles of HFC-254eb in the formed gas (C).)

The evaluation results in each Example are shown in Table 1, and the area ratio (unit: %) in the GC analysis of the formed gas (C) is shown in Table 2. Here, in Table 1, "Pd/C" represents the palladium catalyst supported on coconut shell active carbon, and "Pd/$Al_2O_3$" means a palladium catalyst supported on alumina. Further, in Table 2, compounds having Freon Nos. 1243zf and 263fb are by-products being excessively reduced products, and compounds having Freon Nos. 244eb and 234ea are compounds which can be recycled as reaction intermediates.

TABLE 1

|  | Ex. 1 | Ex. 2 | Ex. 3 | Ex. 4 | Ex. 5 |
|---|---|---|---|---|---|
| Catalyst | Pd/C | Pd/C | Pd/C | Pd/$Al_2O_3$ | Pd/C |
| Ratio ($H_2$/Cl) | 0.43 | 0.54 | 0.625 | 0.5 | 0.75 |
| Conversion ratio X to HFO-1234yf [%] | 56.4 | 61.1 | 56.2 | 48.8 | 66.6 |

(continued)

|  | Ex. 1 | Ex. 2 | Ex. 3 | Ex. 4 | Ex. 5 |
|---|---|---|---|---|---|
| Conversion ratio Y to HCFO-1224yd [%] | 35.5 | 23.8 | 18.5 | 23.3 | 13.1 |
| Conversion ratio Z to HFC-254eb [%] | 6.3 | 11.4 | 15.7 | 25.4 | 18.3 |
| X+Y [%] | 91.9 | 84.9 | 74.7 | 72.2 | 79.7 |

| TABLE 2 | | | | | Unit: % |
|---|---|---|---|---|---|
| Compound | Number | Ex. 1 | Ex. 2 | Ex. 3 | Ex. 4 | Ex.5 |
| $CF_3CF=CH_2$ | 1234yf | 26.70 | 34.21 | 39.30 | 25.29 | 47.66 |
| $CF_3CF=CCl_2$ | 1214ya | 51.65 | 43.96 | 41.46 | 49.38 | 31.20 |
| $CF_3CF=CHCl$ | 1224yd | 16.78 | 13.30 | 9.82 | 10.99 | 8.17 |
| $CF_3CHFCH_2Cl$ | 244eb | 1.49 | 2.00 | 1.00 | 1.46 | 1.45 |
| $CF_3CHFCCl_2$ | 234ea | 0.00 | 0.00 | 0.01 | 0.00 | 0.00 |
| $CF_3CH=CH_2$ | 1243zf | 0.00 | 0.00 | 0.00 | 0.00 | 0.00 |
| $CF_3CH_2CH_3$ | 263fb | 0.18 | 0.00 | 0.18 | 0.16 | 0.21 |
| $CF_3CHFCH_3$ | 254eb | 2.97 | 6.37 | 8.17 | 11.97 | 11.21 |

As shown in Tables 1 and 2, in Examples 1 to 3 wherein a palladium catalyst supported on active carbon was used, and the ratio ($H_2$/Cl) was adjusted to be at most 0.7, formation of the excessively reduced product HFC-254eb as a by-product was lowered as compared with Example 4 wherein a palladium catalyst supported on alumina was used and Example 5 wherein the ratio ($H_2$/Cl) exceeded 0.7. Further, in Examples 1 to 3, the total value of the conversion ratios X and Y was high, and the conversion ratios to the desired product HFO-1234yf and to HCFO-1224yd which can be recycled, were high.

Further, when Examples 1 to 3 were compared, the smaller the ratio ($H_2$/Cl), the higher the effect to suppress formation of by-product HFC-254eb.

INDUSTRIAL APPLICABILITY

HFO-1234yf obtained by the process of the present invention has high purity, as formation of excessively reduced product HFC-254eb as a by-product is suppressed. Therefore, it is useful as e.g. a refrigerant to replace chlorofluorocarbons.

REFERENCE SYMBOLS

| 101: | Reaction apparatus |
|---|---|
| 120A to 120D: | catalyst layers |
| A: | raw material |
| B: | hydrogen |
| C: | nitrogen |

**Claims**

1.  A process for producing 2,3,3,3-tetrafluoropropene, which comprises reacting a raw material compound composed of at least one of 1,1-dichloro-2,3,3,3-tetrafluoropropene and 1-chloro-2,3,3,3-tetrafluoropropene, and hydrogen, in a gas phase in the presence of a catalyst, wherein the catalyst is a palladium catalyst supported on active carbon, and the ratio of the hydrogen to the raw material compound to be introduced to a catalyst layer packed with the palladium-supporting active carbon is at most 0.7 as represented by the ratio of the number of moles of the hydrogen to the number of moles of chlorine atoms in the raw material compound ($H_2$/Cl).

2. The process for producing 2,3,3,3-tetrafluoropropene according to Claim 1, wherein the supported amount of palladium in the palladium-supporting active carbon is from 0.1 to 10 mass% based on the active carbon.

3. The process for producing 2,3,3,3-tetrafluoropropene according to Claim 1 or 2, wherein the active carbon is coconut shell active carbon.

4. The process for producing 2,3,3,3-tetrafluoropropene according to any one of Claims 1 to 3, wherein the ratio of the number of moles of the hydrogen to the number of moles of chlorine atoms in the raw material compound ($H_2$/Cl) is from 0.1 to 0.6.

5. The process for producing 2,3,3,3-tetrafluoropropene according to any one of Claims 1 to 4, wherein the raw material compound is 1,1-dichloro-2,3,3,3-tetrafluoropropene, or a mixture of 1,1-dichloro-2,3,3,3-tetrafluoropropene and 1-chloro-2,3,3,3-tetrafluoropropene wherein the proportion of 1,1-dichloro-2,3,3,3-tetrafluoropropene to the total number of moles of both is at least 50 mol%.

6. The process for producing 2,3,3,3-tetrafluoropropene according to any one of Claims 1 to 5, wherein the 1-chloro-2,3,3,3-tetrafluoropropene is a compound formed as a byproduct in the production of 2,3,3,3-tetrafluoropropene by a reaction of 1,1-dichloro-2,3,3,3-tetrafluoropropene with hydrogen.

7. The process for producing 2,3,3,3-tetrafluoropropene according to any one of Claims 1 to 6, wherein the raw material compound and hydrogen are introduced to a gas inlet portion of the catalyst layer, and hydrogen is introduced from at least one position between the gas inlet portion and a gas outlet portion of the catalyst layer, and wherein the ratio of the total amount of hydrogen including the hydrogen to be introduced to the gas inlet portion of the catalyst layer to the raw material compound is the above-mentioned ratio in the number of moles.

8. A process for producing 2,3,3,3-tetrafluoropropene, which comprises introducing a raw material compound composed of 1,1-dichloro-2,3,3,3-tetrafluoropropene, or 1,1-dichloro-2,3,3,3-tetrafluoropropene and 1-chloro-2,3,3,3-tetrafluoropropene, and hydrogen in such an amount that the ratio of the number of moles of hydrogen to the number of moles of chlorine atoms in the raw material compound ($H_2$/Cl) becomes at most 0.7, to a catalyst layer packed with a palladium catalyst supported on active carbon, to subject them to a gas phase reaction to form 2,3,3,3-tetrafluoropropene, and separating the formed 2,3,3,3-tetrafluoropropene from byproduct 1-chloro-2,3,3,3-tetrafluoropropene to isolate the 2,3,3,3-tetrafluoropropene, wherein recycling of 1-chloro-2,3,3,3-tetrafluoropropene is carried out at least once, said recycling comprising blending the byproduct 1-chloro-2,3,3,3-tetrafluoropropene with 1,1-dichloro-2,3,3,3-tetrafluoropropene to form a fresh raw material compound and carrying out its reaction with the hydrogen.

9. The process for producing 2,3,3,3-tetrafluoropropene according to Claim 8, wherein the supported amount of palladium in the palladium-supporting active carbon is from 0.1 to 10 mass% based on the active carbon.

10. The process for producing 2,3,3,3-tetrafluoropropene according to Claim 8 or 9, wherein hydrogen is introduced in such an amount that the ratio of the number of moles of the hydrogen to the number of moles of chlorine atoms in the raw material compound ($H_2$/Cl) becomes from 0.1 to 0.6, to the catalyst layer.

**Patentansprüche**

1. Verfahren zur Herstellung von 2,3,3,3-Tetrafluorpropen, welches das Umsetzen einer Ausgangsmaterialverbindung, umfassend mindestens eine von 1,1-Dichloro-2,3,3,3-tetrafluorpropen und 1-Chlor-2,3,3,3-tetrafluorpropen, und Wasserstoff in einer Gasphase in der Gegenwart eines Katalysators umfaßt, wobei der Katalysator ein Palladiumkatalysator, getragen auf aktivem Kohlenstoff, ist und das Verhältnis des Wasserstoffs zu der Ausgangsmaterialverbindung, eingeführt zu einer Katalysatorschicht, gepackt mit dem Palladium-tragenden aktiven Kohlenstoff, höchstens 0,7 beträgt, dargestellt durch das Verhältnis der Mol-Zahl des Wasserstoffs zu der Mol-Zahl an Chloratomen in der Ausgangsmaterialverbindung ($H_2$/Cl).

2. Verfahren zur Herstellung von 2,3,3,3-Tetrafluorpropen gemäß Anspruch 1, wobei die getragene Menge an Palladium in dem Palladium-tragenden aktiven Kohlenstoff von 0,1 bis 10 Masse-%, basierend auf dem aktiven Kohlenstoff, beträgt.

3. Verfahren zur Herstellung von 2,3,3,3-Tetrafluorpropen gemäß Anspruch 1 oder 2, wobei der aktive Kohlenstoff Kokosnußschalen-aktiver Kohlenstoff ist.

4. Verfahren zur Herstellung von 2,3,3,3-Tetrafluorpropen gemäß einem der Ansprüche 1 bis 3, wobei das Verhältnis der Mol-Zahl des Wasserstoffs zu der Mol-Zahl an Chloratomen in der Ausgangsmaterialverbindung ($H_2$/Cl) von 0,1 bis 0,6 beträgt.

5. Verfahren zur Herstellung von 2,3,3,3-Tetrafluorpropen gemäß einem der Ansprüche 1 bis 4, wobei die Ausgangsmaterialverbindung 1,1-Dichlor-2,3,3,3-tetrafluorpropen oder ein Gemisch von 1,1-Dichlor-2,3,3,3-tetrafluorpropen und 1-Chlor-2,3,3,3-tetrafluorpropen ist, wobei der Anteil an 1,1-Dichlor-2,3,3,3-tetrafluorpropen zu der Gesamtmol-Zahl von beiden mindestens 50 Mol-% beträgt.

6. Verfahren zur Herstellung von 2,3,3,3-Tetrafluorpropen gemäß einem der Ansprüche 1 bis 5, wobei das 1-Chlor-2,3,3,3-tetrafluorpropen eine Verbindung ist, gebildet als ein Nebenprodukt in der Herstellung von 2,3,3,3-Tetrafluorpropen durch eine Umsetzung von 1,1-Dichlor-2,3,3,3-tetrafluorpropen mit Wasserstoff.

7. Verfahren zur Herstellung von 2,3,3,3-Tetrafluorpropen gemäß einem der Ansprüche 1 bis 6, wobei die Ausgangsmaterialverbindung und Wasserstoff zu einem Gaseinlaßabschnitt der Katalysatorschicht eingeführt werden und Wasserstoff von mindestens einer Stelle zwischen dem Gaseinlaßabschnitt und einem Gasauslaßabschnitt der Katalysatorschicht eingeführt wird, und wobei das Verhältnis der Gesamtmenge an Wasserstoff einschließlich des Wasserstoffs, der zu dem Gaseinlaßabschnitt der Katalysatorschicht eingeführt wird, zu der Ausgangsmaterialverbindung das vorstehende Verhältnis in den Mol-Zahlen ist.

8. Verfahren zur Herstellung von 2,3,3,3-Tetrafluorpropen, welches das Einführen einer Ausgangsmaterialverbindung, umfassend 1,1-Dichlor-2,3,3,3-tetrafluorpropen oder 1,1-Dichlor-2,3,3,3-tetrafluorpropen und 1-Chlor-2,3,3,3-tetrafluorpropen, und Wasserstoff in einer solchen Menge, daß das Verhältnis der Mol-Zahl an Wasserstoff zu der Mol-Zahl an Chloratomen in der Ausgangsmaterialverbindung ($H_2$/Cl) höchstens 0,7 wird, zu einer Katalysatorschicht, gepackt mit einem Palladiumkatalysator, getragen auf aktivem Kohlenstoff, um diese einer Gasphasenreaktion zu unterwerfen, um 2,3,3,3-Tetrafluorpropen zu bilden, und Abtrennen des gebildeten 2,3,3,3-Tetrafluorpropen vom Nebenprodukt 1-Chlor-2,3,3,3-tetrafluorpropen, um das 2,3,3,3-Tetrafluorpropen zu isolieren, wobei das Rückführen von 1-Chlor-2,3,3,3-tetrafluorpropen mindestens einmal durchgeführt wird, wobei das Rückführen das Mischen des Nebenprodukts 1,1-Chlor-2,3,3,3-tetrafluorpropen mit 1,1-Dichlor-2,3,3,3-tetrafluorpropen umfaßt, um eine frische Ausgangsmate-rialverbindung zu bilden und dessen Umsetzung mit dem Wasserstoff durchzuführen, umfasst.

9. Verfahren zur Herstellung von 2,3,3,3-Tetrafluorpropen gemäß Anspruch 8, wobei die getragene Menge an Palladium in dem Palladium-tragenden aktiven Kohlenstoff von 0,1 bis 10 Masse-%, basierend auf dem aktiven Kohlenstoff, beträgt.

10. Verfahren zur Herstellung von 2,3,3,3-Tetrafluorpropen gemäß Anspruch 8 oder 9, wobei Wasserstoff in einer solchen Menge, daß das Verhältnis der Mol-Zahlen des Wasserstoffs zu den Mol-Zahlen an Chloratomen in der Ausgangsmaterialverbindung ($H_2$/Cl) von 0,1 bis 0,6 wird, zu der Katalysatorschicht eingeführt wird.

**Revendications**

1. Procédé pour produire du 2,3,3,3-tétrafluoropropène, qui comprend la réaction d'un composé matière première composé d'au moins l'un parmi le 1,1-dichloro-2,3,3,3-tétrafluoropropène et le 1-chloro-2,3,3,3-tétrafluoropropène, et d'hydrogène, en phase gazeuse en présence d'un catalyseur, dans lequel le catalyseur est un catalyseur au palladium supporté sur du charbon actif, et le rapport de l'hydrogène sur le composé matière première devant être introduit dans une couche de catalyseur garnie du charbon actif supportant du palladium est d'au plus 0,7, tel que représenté par le rapport du nombre de moles d'hydrogène sur le nombre de moles d'atomes de chlore dans le composé matière première ($H_2$/Cl).

2. Procédé pour produire du 2,3,3,3-tétrafluoropropène selon la revendication 1, dans lequel la quantité supportée de palladium dans le charbon actif supportant du palladium est de 0,1 à 10 % en masse par rapport au charbon actif.

**3.** Procédé pour produire du 2,3,3,3-tétrafluoropropène selon la revendication 1 ou 2, dans lequel le charbon actif est du charbon actif de coque de noix de coco.

**4.** Procédé pour produire du 2,3,3,3-tétrafluoropropène selon l'une quelconque des revendications 1 à 3, dans lequel le rapport du nombre de moles d'hydrogène sur le nombre de moles d'atomes de chlore dans le composé matière première ($H_2$/Cl) est de 0,1 à 0,6.

**5.** Procédé pour produire du 2,3,3,3-tétrafluoropropène selon l'une quelconque des revendications 1 à 4, dans lequel le composé matière première est le 1,1-dichloro-2,3,3,3-tétrafluoropropène, ou un mélange de 1,1-dichloro-2,3,3,3-tétrafluoropropène et de 1-chloro-2,3,3,3-tétrafluoropropène dans lequel la proportion de 1,1-dichloro-2,3,3,3-tétrafluoropropène par rapport au nombre total de moles des deux est d'au moins 50 % en moles.

**6.** Procédé pour produire du 2,3,3,3-tétrafluoropropène selon l'une quelconque des revendications 1 à 5, dans lequel le 1-chloro-2,3,3,3-tétrafluoropropène est un composé formé en tant que sous-produit dans la production de 2,3,3,3-tétrafluoropropène par la réaction de 1,1-dichloro-2,3,3,3-tétrafluoropropène avec de l'hydrogène.

**7.** Procédé pour produire du 2,3,3,3-tétrafluoropropène selon l'une quelconque des revendications 1 à 6, dans lequel le composé matière première et l'hydrogène sont introduits au niveau d'une partie d'entrée de gaz de la couche de catalyseur, et l'hydrogène est introduit depuis au moins une position entre la partie d'entrée de gaz et une partie de sortie de gaz de la couche de catalyseur, et dans lequel le rapport de la quantité totale d'hydrogène, y compris l'hydrogène devant être introduit dans la partie d'entrée de gaz de la couche de catalyseur, sur le composé matière première, est le rapport susmentionné en nombre de moles.

**8.** Procédé pour produire du 2,3,3,3-tétrafluoropropène, qui comprend l'introduction d'un composé matière première composé de 1,1-dichloro-2,3,3,3-tétrafluoropropène ou de 1,1-dichloro-2,3,3,3-tétrafluoropropène et de 1-chloro-2,3,3,3-tétrafluoropropène, et d'hydrogène, en une quantité telle que le rapport du nombre de moles d'hydrogène sur le nombre de moles d'atomes de chlore dans le composé matière première ($H_2$/Cl) devienne d'au plus 0,7, dans une couche de catalyseur garnie d'un catalyseur au palladium supporté sur du charbon actif, la soumission de ceux-ci à une réaction en phase gazeuse pour former du 2,3,3,3-tétrafluoropropène, et la séparation du 2,3,3,3-tétrafluoropropène formé, d'avec le sous-produit 1-chloro-2,3,3,3-tétrafluoropropène pour isoler le 2,3,3,3-tétrafluoropropène, dans lequel le recyclage du 1-chloro-2,3,3,3-tétrafluoropropène est effectué au moins une fois, ledit recyclage comprenant le mélange du sous-produit 1-chloro-2,3,3,3-tétrafluoropropène avec du 1,1-dichloro-2,3,3,3-tétrafluoropropène pour former un composé matière première frais et la mise en oeuvre de sa réaction avec l'hydrogène.

**9.** Procédé pour produire du 2,3,3,3-tétrafluoropropène selon la revendication 8, dans lequel la quantité supportée de palladium dans le charbon actif supportant du palladium est de 0,1 à 10 % en masse par rapport au charbon actif.

**10.** Procédé pour produire du 2,3,3,3-tétrafluoropropène selon la revendication 8 ou 9, dans lequel l'hydrogène est introduit dans la couche de catalyseur en une quantité telle que le rapport du nombre de moles d'hydrogène sur le nombre de moles d'atomes de chlore dans le composé matière première ($H_2$/Cl) devienne de 0,1 à 0,6.

## Fig. 1

**EP 2 586 758 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008060614 A